(19) Europäisches Patentamt
European Patent Office
Office européen des brevets

(11) **EP 3 533 442 B1**

(12) **EUROPEAN PATENT SPECIFICATION**

(45) Date of publication and mention
of the grant of the patent:
**22.07.2020 Bulletin 2020/30**

(51) Int Cl.:
*A61K 9/127* (2006.01)  *A61K 9/19* (2006.01)
*A61K 47/10* (2017.01)  *A61K 47/12* (2006.01)
*A61K 47/18* (2017.01)  *A61K 47/24* (2006.01)
*A61K 47/28* (2006.01)  *A61K 31/12* (2006.01)
*A61K 36/9066* (2006.01)

(21) Application number: **18386004.8**

(22) Date of filing: **01.03.2018**

(54) **PROCESS FOR THE PRODUCTION OF LIPIDIC VEHICLES**

VERFAHREN ZUR HERSTELLUNG VON LIPIDISCHEN TRÄGERN

PROCÉDÉ DE PRODUCTION DE VÉHICULES LIPIDIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.02.2018 GR 20180100082**

(43) Date of publication of application:
**04.09.2019 Bulletin 2019/36**

(73) Proprietor: **Nikolaos A. Fikioris Kai Sia Etaireia
Periorismenis Efthinis with distinguishing title
In Touch Health E.P.E.
10671 Athens (GR)**

(72) Inventors:
• **Naziris, Nikolaos**
 **Athens (GR)**
• **Pippa, Anastasia-Georgia**
 **Athens (GR)**
• **Demetzos, Costas**
 **Piraeus (GR)**

(74) Representative: **Yazitzoglou, Evagelia S. et al
Dr. Helen G. Papaconstantinou and Partners
2, Coumbari Street
Kolonaki
106 74 Athens (GR)**

(56) References cited:
**EP-A1- 2 253 308**  **EP-A2- 0 130 577**
**WO-A1-88/06442**  **WO-A1-91/10422**
**WO-A1-2005/084641**  **US-A1- 2005 260 260**
**US-A1- 2008 274 172**  **US-A1- 2010 239 521**

• **DATABASE WPI Section Ch, Week 201750
Thomson Scientific, London, GB; Class A96, AN
2017-39003N XP002785024, JIANG Z; LI C; SHEN
X; WANG D; ZHAN H: "Blank liposome useful for
preparing liposomes of the loaded active
substance, comprises raw material composition
of blank liposomes is to partially replace the
cholesterol in the ordinary blank liposomes with
glycyrrhizic compound", -& CN 106 692 056 A
(SHANGHAI GINPOSOME PHARMATECH CO
LTD) 24 May 2017 (2017-05-24)**

Remarks:
The file contains technical information submitted after
the application was filed and not included in this
specification

## Description

## TECHNICAL FIELD

The present invention relates to a process for producing lipidic vehicles, such as liposomes.

## BACKGROUND ART

Lipidic bioactive ingredient delivery systems

Lipidic-based bioactive ingredient delivery systems have shown great potentials in the delivery of poorly water-soluble bioactive ingredients, such as drugs, primarily lipophilic, with several successfully marketed products. Lipidic vehicle structures (e.g. double layer, cubic, hexagonal etc.) depend on the phospholipid/lipid concentration that takes part in the process and on their geometry, which is related to their chemical structure (D. Papahadjopoulos, J.C. Watkins, Phospholipid model membrane, Permeability properties of hydrated liquid crystals, Biochim. Biophys. Acta 135, 639 (1967); J. Milhaud, New insights into water-phospholipid model membrane interactions, Biochim. Biophys. Acta 1663, 19 (2004); D.D. Lasic, et al., Spontaneous vesiculation, Adv. Colloid. Interface Sci. 89-90, 337 (2001)). For this reason, they can entrap hydrophobic, hydrophilic or even amphiphilic bioactive ingredients. Pre-dissolving bioactive ingredients in lipids, surfactants or mixtures of lipids and surfactants omits the dissolution step, which is a potential rate limiting factor to the oral absorption of poorly water-soluble bioactive ingredients and consequently, to their effectiveness. However, lipids not only vary in structure and physiochemical properties, but also in their digestibility and absorption pathway; therefore, the selection of lipid excipients and dosage form has a profound effect on the biopharmaceutical and pharmacokinetic aspects of bioactive ingredients, including absorption and distribution inside the organism. These effects can be observed both in vitro and in vivo (C. Demetzos, Pharmaceutical Nanotechnology: Fundamentals and practical applications. Springer ISBN 978-981-10-0791-0 (2016)).

Liposomes

Liposomes are closed, pseudo-spherical structures that are consisted of one or more lipid bilayers, entrapping water media inside them and are characterized as thermodynamically unstable colloidal dispersions (D. Papahadjopoulos, J.C. Watkins, Phospholipid model membrane, Permeability properties of hydrated liquid crystals, Biochim. Biophys. Acta 135, 639 (1967); J. Milhaud, New insights into water-phospholipid model membrane interactions, Biochim. Biophys. Acta 1663, 19 (2004); D.D. Lasic, et al., Spontaneous vesiculation, Adv. Colloid. Interface Sci. 89-90, 337 (2001); V. Guida, Thermodynamics and kinetics of vesicles formation processes, Adv. Colloid. Interface Sci. 161(1-2), 77 (2010); C. Puglia, F. Bonina, Lipid nanoparticles as novel delivery systems for cosmetics and dermal pharmaceuticals, Expert Opin. Drug Deliv. 9,429 (2012)). Lipid bilayers are consisted mainly of phospholipids and cholesterol, without excluding the use of other biomaterials, e.g. polymers, as liposomal structural units. Phospholipids, due to their amphiphilic nature, orientate and self-assemble inside water environment, so that their polar heads end up towards the water medium, while their lipophilic hydrocarbon chains are protected from the water molecule connection, by developing hydrophobic interactions amongst them. This behavior is defined by their geometric characteristics and more specifically, by their critical packing parameter:

$$C_{PP} = \frac{V}{a_0 l_c}$$

where V is the surfactant tail volume, $l_c$ is the carbon chain length and ao is the equilibrium area per molecule at the aggregate surface. For example, molecules with packing parameter $1/2 < CPP < 1$ have a truncated cone shape and self-assemble into bilayer vesicles (J.N. Israelachvili et al., Theory of self-assembly of hydrocarbon amphiphiles into micelles and bilayers, J. Chem. Soc., Far. Trans. 2: Mol. Chem. Phys., 72, 1525 (1976); R. Nagarajan, Molecular Packing Parameter and Surfactant Self-Assembly: The Neglected Role of the Surfactant Tail, Langmuir, 18, 31 (2002)).

On the liposomal surface, small molecules or macromolecules can be attached, e.g. polymers, peptides, receptor-specific ligands and antibodies; those change the physicochemical properties of the surface. This fact is very important and defines the nanosystem functionality, which depends on its physicochemical characteristics immediately after production, as well as physical stability over time.

There are several types of liposomes, based on their size, number of bilayers, charge, surface properties and functionality (D. Papahadjopoulos, et al., Sterically stabilized liposomes: Improvements in pharmacokinetics and antitumor therapeutic efficacy, Proc. Natl. Acad. Sci. USA 88, 11460 (1991); M. Riaz, Liposome Preparation Method, J.

Pharm. Sci. 19 65 (1996); Y.P. Patil, S. Jadhav, Novel methods for liposome preparation. Chem Phys. Lipids. 177:8-18 (2014)). During the past years, liposome-related research flourished, as transport and delivery vehicles for therapeutic purposes (i.e. delivery of drugs/pharmaceuticals and biopharmaceuticals) were developed. Liposomes exhibit a number of important biological properties, some of which are the capability for inclusion of both hydrophilic and hydrophobic drug molecules, the provision of protection for these molecules, the ability for delivery into specific cells or cellular compartments, but also the biocompatibility that characterizes them, along with the flexible customization of their physicochemical and biological properties.

Due to their double nature (liposomal bilayer and hydrophilic core), liposomes can be utilized as carriers for both lipophilic and hydrophilic bioactive ingredients. Depending on their nature, various bioactive ingredients can be placed inside the lipid bilayer or in the hydrophilic section of liposomes. In the first case, the bioactive ingredient is incorporated into the bilayers of liposomes, while in the second case, the bioactive ingredient is encapsulated inside the aqueous interior of liposomes. Changing their lipid composition (phospholipids and generally lipids of different structure), $\zeta$-potential, size and size distribution has been reported as crucial and could define liposomal behavior in vivo and in vitro (C. Demetzos, Pharmaceutical Nanotechnology: Fundamentals and practical applications. Springer ISBN 978-981-10-0791-0 (2016)).

The lipidic vehicles' production through self-assembly and structural organization, depending on the biomaterials' structural and geometrical characteristics, physicochemical properties and energy content, is an important fact that defines the development of final pharmaceutical products. It is directly related to the vehicles' physicochemical characteristics, physical stability, bioactive ingredient entrapment ability, release rate and efficacy, according to their absorption, distribution, metabolism, and excretion (ADME) profile.

## Processes of preparation of liposomes and other lipid-based nanoparticles

Methods for producing liposomes include various techniques, such as the reverse-phase evaporating method, sonication method, extrusion method, French press method, homogenization method, ethanol injection method, dehydration-rehydration method, of which one typical technique is the Bangham method (thin-film hydration method) (D. Papahadjopoulos, et al., Sterically stabilized liposomes: Improvements in pharmacokinetics and antitumor therapeutic efficacy, Proc. Natl. Acad. Sci. USA 88, 11460 (1991); M. Riaz, Liposome Preparation Method, J. Pharm. Sci. 19 65 (1996); Y.P. Patil, S. Jadhav, Novel methods for liposome preparation. Chem Phys. Lipids. 177:8-18 (2014)). According to the Bangham method, a suspension containing liposomes is obtained as follows: at least one phospholipid is dissolved in an organic solvent, such as chloroform and the solution is placed inside a vessel, such as a roundbottom flask; then, by evaporating off chloroform, lipid membrane is temporarily formed at the bottom of the vessel, on which an aqueous solution, such as a buffer, is added and the vessel is mixed. In this way, multi-lamellar vesicles (MLVs) are produced and further processed by size-reducing and homogenizing techniques, in order to obtain liposomes, which are small unilamellar vesicles (SUVs).

More recently, a new categorization of liposome preparation techniques took place, as described below. The conventional methods of preparing giant unilamellar vesicles (GUVs) are: gentle hydration of a phospholipid film, electroformation (the phospholipid film is deposited on electrodes and subsequently hydrated for a couple of hours in the presence of an electric field) and the freeze-thaw cycles. For the preparation of MLVs, the following protocols are used: hydration of phospholipid film under hydrodynamic flow (vacuum), solvent-spherule method and hydration of proliposomes. SUVs and large unilamellar vesicles (LUVs) are prepared by the following methods: reserve-phase evaporation, injection of organic solvent with dissolved phospholipids into an aqueous phase, detergent dialysis and reduction of size and lamellarity of MLVs. For scale up, microfluidics is the method of preparation of liposomes and lipid-based systems. Microfluidics involves fluid flow in channels having cross-sectional dimensions, typically in the range of 5-500 nm. In the last decade, several novel microfluidics-based techniques have been developed to produce liposomes. The features of microfluidic systems that can be used to the advantage of liposome production include the ability to accurately dispense nanoliter volumes, precise control over the position of the interface, diffusion-dominated axial mixing and continuous mode of operation at low volumes. In addition, US20100239521 discloses a process for the production of a complex of a bioactive ingredient in a polymeric or lipid carrier. The process comprises providing a complexation zone supplied with an aqueous medium containing the carrier material, stirring and heating the medium, adding the bioactive ingredient to the aqueous medium and recovering the carrier complex of the bioactive ingredient. WO2005084641 discloses a process similar to that of US20100239521, in which in addition, an inert gas passes through the aqueous medium.

US 2008/274172 A1 relates to a method for preparing liposomes with at least two uniformly-distributed phospholipids, without using organic solvents.

The prior art processes for the production of lipidic carriers have a number of disadvantages. Thus, in many of those processes, chlorinated and other volatile organic solvents are used. However, these solvents represent a safety and health hazard and it is desirable to avoid their use. Furthermore, many prior art processes are complex and/or require considerable amount of resources, time and effort. In addition, the size and polydispersity index of the carriers achieved by most prior art processes is not satisfactory and for this reason, additional steps, such as ultrafiltration, are needed.

The present invention provides a process for the production of lipidic vehicles which successfully addresses the disadvantages of the prior art processes.

## SUMMARY OF THE INVENTION

The present invention provides the process of claim 1 for the production of lipidic vehicles, wherein the process comprises stirring a mixture of a lipid and a promoter in a liquid medium comprising water and a liquid polyol, heating the mixture in two steps, wherein the temperature of the mixture in the second step is higher than the temperature in the first step and allowing the mixture to cool down to room temperature.

The process utilizes, in addition to the mechanical shock caused by the stirring, a thermal shock caused by the temperature increase in the second step of heating. This leads to the formation of lipidic vehicles and liposomes with desirable characteristics, such as size and polydispersity index (PDI).

The process enables the production of lipidic vehicles and liposomes without using chlorinated or other volatile organic solvents. Furthermore, the process does not require the use of sonication or centrifugation. In addition, the process does not require the use of reduced pressure.

## BRIEF DESCRIPTION OF THE DRAWINGS

Example 9 is not according to the invention.

Figure 1 shows the distribution and peak analysis by A) intensity ($D_I$), B) volume (Dv) and C) number ($D_N$) of liposomes with 20% glycerin of Example 1.

Figure 2 shows the distribution and peak analysis by A) intensity ($D_I$), B) volume (Dv) and C) number ($D_N$) of liposomes with 1.8 cholesterol molar ratio of Example 2.

Figure 3 shows the size stability of liposomes of Example 3.

Figure 4 shows the PDI stability of liposomes of Example 3.

Figure 5 shows the size stability of liposomes of Example 6.

Figure 6 shows the PDI stability of liposomes of Example 6.

Figure 7 shows the distribution and peak analysis by A) intensity ($D_I$), B) volume (Dv) and C) number ($D_N$) of liposomes with 20% glycerin of Example 9.

## DETAILED DESCRIPTION OF THE INVENTION

The present invention provides a process for the production of lipidic vehicles, preferably liposomes, comprising the following steps:

a) providing a mixture of an amphiphilic lipid and a promoter in a liquid medium comprising water and a liquid polyol, wherein the promoter is selected from dipalmitoylphosphatidylglycerol, dioleoyltrimethylammoniumpropane, cholesterol, fatty acids, fatty acid amines, poloxamers, polyethylene glycol with molecular weight higher than 600 and mixtures thereof, and the liquid polyol is selected from glycerine, propylene glycol, polyethylene glycol with molecular weight lower than 600 and mixtures thereof,

b) stirring and heating the mixture in a first heating step at 30-80°C,

c) stirring and heating the mixture in a second heating step at a temperature 10-50°C higher than the temperature of the first heating step and

d) allowing the mixture to cool down to room temperature.

The invention is defined by the claims. Any subject-matter falling outside the scope of the claims is provided for information purposes only.

A "lipidic vehicle" is herein defined as a bioactive ingredient (e.g. drug, nutraceutical, cosmeceutical etc.) delivery system primarily of lipidic nature, which is comprised primarily of amphiphilic lipids, such as phospholipids or non-ionic surfactants. It may also contain small amounts of self-assembly promoters, such as lipid amphiphilic or lipophilic biomaterials (e.g. sterols) and non-lipid amphiphilic or lipophilic biomaterials (e.g. polymers).

A "liposome" is herein defined as a subcategory of lipidic vehicles and more specifically, a sphere-like vesicle of size up to 800nm, consisting of at least one lipid bilayer and comprising primarily phospholipids.

An "amphiphilic lipid" is herein defined as a molecule of amphiphilic nature that contains a lipidic part and has a packing parameter between 1/2 and 1, rendering it able to form lipidic vehicles and preferably liposomes. Examples of natural and/or synthetic amphiphilic lipids according to the present invention include phospholipids, such as hydrogenated soy phosphatidylcholine (HSPC), egg phosphatidylcholine (eggPC), dipalmitoylphosphatidylcholine (DPPC), distearoylphosphatidylcholine (DSPC), dimyristoylphosphatidylcholine (DMPC), dioleoylphosphatidylcholine (DOPC), dipalmitoylphosphatidylethanolamine (DPPE), distearoylphosphatidylethanolamine (DSPE), dioleoylphosphatidylethanolamine (DOPE), ether lipids, cardiolipins or any type of fatty acid- or headgroup-modified phospholipid that forms vesicles. They also include sphingolipids, such as ceramides and adjuvant lipids, such as dimethyldioctadecylammonium (DDA). Ionic or non-ionic surfactants, such as sorbitan lipids and polysorbates, which form niosomes, also belong in this category. Mixtures of all the above mentioned molecules are possible.

A "promoter" is herein defined as a molecule or substance that is added in low amounts in the vehicles, facilitates the self-assembly process of the above mentioned bulk amphiphilic lipids and improves the final vehicles' physicochemical, thermodynamic and biophysical profile. A promoter, as fluidity regulator, will stabilize the vehicle structure and make it more functional. Examples of promoters include dipalmitoylphosphatidylglycerol (DPPG) and dioleoyltrimethylammoniumpropane (DOTAP), sterols, such as cholesterol, charged or not charged amphiphilic molecules, fatty acids and fatty acid amines, such as stearylamine, as well as the PEGylated analogues of the above. Polymers, such as stimuli-responsive/sensitive polymers, poloxamers and polyethylene glycols (PEGs) with molecular weight higher than 600 also belong in this category. Mixtures of all the above mentioned promoters are also possible. Preferably, the promoter is stearylamine.

The term "liquid polyol" is herein defined as a water-soluble organic compound, which is liquid at 25°C and comprises multiple hydroxyl groups. The liquid polyol drives/enhances the hydration process of the amphiphilic lipid(s), facilitating their self-assembly in smaller vehicles and managing the thermodynamic content of the lipidic vehicles and liposomes. In cases where the lipidic vehicles are subjected to lyophilization, the liquid polyol also plays a key role in the physical stability and cryo-protection of lipidic vehicles during lyophilization and reconstitution process. Liquid polyols according to the present invention include glycerine (glycerol), propylene glycol (PG), PEGs with molecular weight lower than 600 and mixtures thereof. Preferably, the liquid polyol is glycerine.

A "bioactive ingredient" is herein defined as an ingredient that has an effect on a living organism, tissue or cell. Examples are drugs or active pharmaceutical ingredients (APIs), nutraceuticals, cosmeceuticals etc. More than one bioactive ingredients may be encapsulated or incorporated in the lipidic vehicles and liposomes and delivered inside the organism via oral (per os), intravenous (i.v.) or other way of administration.

The present approach is suitable for preparing various lipidic vehicles and liposomes in the absence of chlorinated and other volatile organic solvents. Examples of these vehicles are liposomes, mixed/chimeric liposomes, niosomes, mixed/chimeric niosomes and polymer-grafted liposomes. Preferably, the lipidic vehicles produced by the process of the present invention are liposomes.

Generally, the process depends on the fluidity/mobility of liquid crystalline materials above their phase transition temperature ($T_m$), as well as their rigidity below that point. As a result, it is suitable for producing structures composed e.g. from phospholipids. Specifically, the lipidic vehicles are formed due to hydration from the aqueous medium molecules and co-solvent molecules, mechanical shock, resulting from the stirring process and heating shock, coming from temperature increase.

Preferably, the total concentration of dispersed amphiphilic lipid(s) and promoter(s) in the liquid medium ranges between 5-100 mg/mL. The term "mg/mL" refers to mg of the substances in question per mL of the liquid medium. The term "liquid medium" refers to the system of the bulk aqueous medium (e.g. water) and co-solvent(s) (i.e. liquid polyol(s)).

Preferably, the polyol concentration is up to 30% v/v of the liquid medium. The term "% v/v" refers to ml of polyol per 100ml of the liquid medium. More preferably, the polyol concentration is 10-30% v/v of the volume of the liquid medium.

Preferably, the total weight of the promoter(s) ranges between 0.1-20.0% w/w of the total weight of the amphiphilic lipid(s). The term "% w/w" refers to mg of promoter(s) per 100mg of the amphiphilic lipid(s).

In the first heating step of the process of the present invention, the mixture of amphiphilic lipid(s) with the promoter(s) in the liquid medium is heated above the amphiphilic lipids' main transition temperature ($T_m$), namely at 30-80°C, while stirred. In the second heating step, the temperature of the mixture is increased by 10-50°C and the mixture is further stirred. Preferably, the stirring speed and temperature are kept stable during each step. Preferably, in the second heating step the temperature of the mixture is increased by 20-40°C, compared with the temperature of the first heating step. More preferably, in the second heating step, the temperature of the mixture is increased by 25-35°C, compared with the temperature of the first heating step. Preferably, the stirring and heating of the first heating step is carried out for 0.5-2 hours. Preferably, the stirring and heating of the second heating step is carried out for 0.5-4 hours. Preferably, the stirring speed in both heating steps is 400-1000rpm. In the final step of the process, the mixture is cooled down to room temperature (25°C), preferably with cooling rate not greater than 5°C/min. The level of temperature, the stirring speed and the duration and rate of heating or cooling may affect the physicochemical properties of the prepared lipidic vehicles and liposomes. However, the adjustment of these parameters lies within the common knowledge of a person skilled in the art.

Depending on their composition, the lipidic vehicles produced by the process of the present invention reach approximately 100nm with 0.200 polydispersity index (PDI), without size reduction or further processing. This represents a great advantage over the processes of the prior art which utilize extrusion through filters or similar methods. It is noted that the physicochemical properties of produced vehicles depend on the $T_m$ of the utilized amphiphilic lipid. For example, HSPC has a very high melting temperature of about 52°C, while eggPC has a much lower transition temperature 23°C and is in liquid crystalline phase in room temperature. The method produces, in few and simple steps, small and uniform in diameter lipidic vehicles. Those are suitable for oral (per os), as well as for intravenous (i.v.) and other types of administration.

Although it is not necessary, the lipidic vehicles produced according to the present invention can be subjected to methods for size reduction, including sonication, homogenization and extrusion. In cases of low polyol content and in cases of compositions that result in vehicles with large diameters and high polydispersity, the vehicles can be improved by such methods, in order to utilize them in per os, i.v. or other types of administration. One cycle of extrusion is enough to improve vehicles in this approach, which means low energy consumption and consequently, low economic cost.

Lyophilization process can be applied to all vehicles produced according to the present invention, due to the cryo-protection and lyo-protection of polyols. The lyophilized products remain stable for long periods. In polyol concentrations above 10%, the addition of liquid medium of equal volume with the initial leads to resuspension of the lipidic vehicles and liposomes and restoration of their initial physicochemical state and properties i.e. particle size, polydispersity and $\zeta$-potential.

In the case of per os administration, sweeteners or mixtures thereof, such as sugars, sugar substitutes and flavors can be added to the liquid medium, during step a), b) c) or d) of the claimed process and serve as sweetening factors, as well as facilitate lyophilization, as they serve as cryo- and lyo-protectants. Their sweetening property is maintained after lyophilization and reconstitution. Examples of sugars are glucose, fructose and sucrose, while some sugar substitutes are aspartame and saccharin.

The lipidic vehicles produced according to the present invention can be loaded with various lipophilic and/or hydrophilic natural and/or synthetic bioactive ingredients, such as drugs, nutraceuticals and cosmeceuticals. In such a case, the bioactive ingredient(s) can be added to the liquid medium during any step of the claimed process, preferably during a), b) or c). Encapsulation of hydrophilic, incorporation of lipophilic or distribution of amphiphilic ingredients is achieved inside the vehicles. However, when the vehicles undergo lyophilization, the bioactive ingredient(s) may also be added to the lyophilized product, where the above mentioned phenomena occur during reconstitution. A simple mixing of the prepared vehicles with the bioactive ingredient(s) and subsequent vortexing or sonication will provide limited amount of encapsulation or incorporation or distribution. Better results can be obtained with freeze-thaw process. Examples of bioactive ingredients include drugs, nutraceuticals and cosmeceuticals, as well as mixtures therefore. Those could be classic pharmaceutical drugs or APIs, genes, proteins, peptides, hormones, such as insulin, antibodies, as well as flavonoids, polyphenols, carotenoids, carbohydrates, prebiotics, curcuminoids, such as curcumin, which are ingredients of the turmeric (curcuma longa) extract, glutathione, lipoic acid and vitamins of the complexes B, C, D and K. Essential oils and extracts also belong in this category of bioactive ingredients. The bioactive ingredients do not need to be stabilized before incorporation and/or encapsulation into the vehicles and remain stable after the formulation process. Moreover, the cooperativity between the utilized biomaterials facilitates their incorporation and/or encapsulation, a process that does not require the utilization of metal ingredients etc. The process temperature must be appropriate for the bioactive ingredient, not affecting its stability and as a result, appropriate amphiphilic lipid(s) must be utilized that melt in that temperature. Coadministration of more than one bioactive ingredients in the same formulation of lipidic vehicles is achievable, especially due to the ability of these particles to entrap ingredients in different compartments e.g. encapsulation of hydrophilic ones in the aqueous core and incorporation of lipophilic or hydrophobic ones in the lipid bilayer. The administration of empty vehicles is also of value for some purposes, for example in Cosmetics, where their composition may provide hydration or protection.

The process of the present invention can be easily scaled-up and utilized in industry, with or without the use of size reduction techniques, due to simple method parameters, accurate and repeatable results, time efficiency, low cost, absence of chlorinated and volatile organic solvents, safety and low temperature.

## EXAMPLES

The present invention is illustrated by the following examples:

Example 1

Preparation of HSPC:stearylamine liposomes

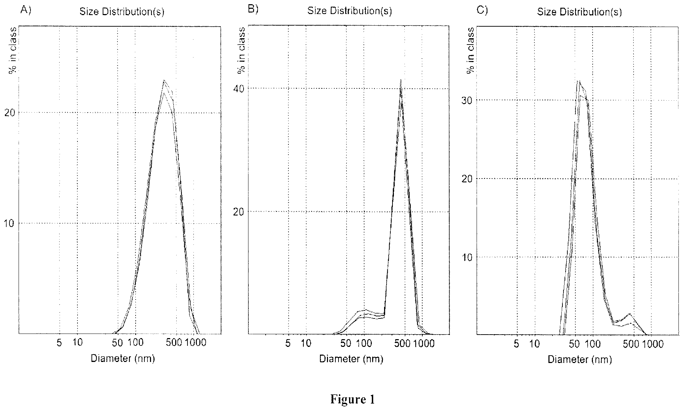

HSPC (30.0mg) and stearylamine (0.3mg) of molar ratio 9:0.25 were weighted and placed inside a large spherical flask. 3mL of purified water with dissolved glycerine 20/15/10% v/v were added to the flask and the mixture was vortexed for a brief period. A magnet was placed inside and the mixture was heated at 60°C, while stirred at 700rpm, for a 1hour period, in a silicon oil bath. Then, the suspension was heated at 90°C for 1 hour, at the same stirring. The formed suspension was left to cool down to room temperature with a rate 3°C/min and a sample of 50uL was extracted, which was diluted with 2950uL of HPLC-grade water and measured with photon correlation spectroscopy (PCS), to calculate the liposome size, polydispersity and $\zeta$-potential. Then, the suspension was heated at 90°C for 1hour, at 700rpm and after cooling, another sample of 50uL was measured. The results for the prepared liposomes are presented in Table 1 and the size distribution by intensity, volume and number of particles with 20% glycerin is presented in Figure 1, where for each diagram, there are three curves, which in this case are very close to each other. The peak analysis areas and mean values of the three methods are given below:

Peak Analysis by intensity

| Peak | Area | Mean | Width |
|---|---|---|---|
| 1 | 100.0 | 336.3 | 520.3 |

Peak Analysis by volume

| Peak | Area | Mean | Width |
|---|---|---|---|
| 1 | 16.7 | 123.3 | 102.3 |
| 2 | 83.3 | 458.0 | 347.3 |

Peak Analysis by number

| Peak | Area | Mean | Width |
|---|---|---|---|
| 1 | 96.5 | 77.3 | 64.1 |
| 2 | 3.5 | 441.1 | 178.5 |

Table 1

| System | Molar ratio | Glycerin Concentration (% v/v) | Hours at 90°C | $Z_{Ave}$ (nm) | SD | PDI | SD | z-pot (mV) | SD |
|---|---|---|---|---|---|---|---|---|---|
| HSPC: stear | 9:0.25 | 20% | 1 | 275.5 | 4.1 | 0.349 | 0.003 | | |
| HSPC: stear | 9:0.25 | 20% | 2 | 230.5 | 4.6 | 0.272 | 0.017 | 51.5 | 2.9 |
| HSPC: stear | 9:0.25 | 15% | 1 | 366.8 | 10.8 | 0.616 | 0.078 | | |
| HSPC: stear | 9:0.25 | 15% | 2 | 289.2 | 10.0 | 0.432 | 0.016 | 52.8 | 2.2 |
| HSPC: stear | 9:0.25 | 10% | 1 | 346.9 | 9.4 | 0.567 | 0.080 | | |
| HSPC: stear | 9:0.25 | 10% | 2 | 290.4 | 9.7 | 0.484 | 0.055 | 54.5 | 0.6 |

Example 2

Preparation of eggPC:cholesterol:stearylamine liposomes

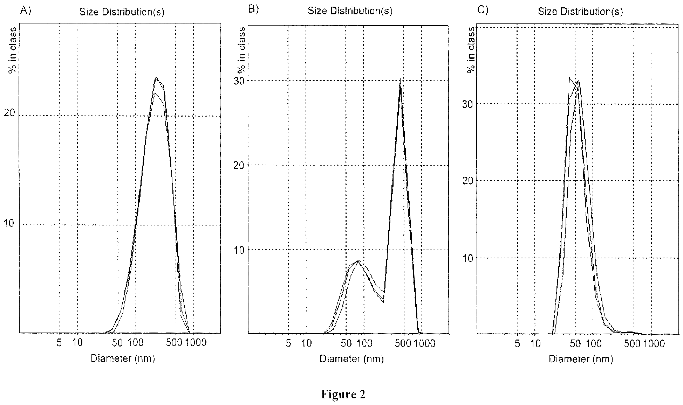

EggPC (30.0mg), cholesterol (0.8/3.0mg) and stearylamine (0.3mg) of molar ratio 9:0.5:0.25 or 9:1.8:0.25 were weighted and placed inside a large spherical flask. 3mL of purified water with dissolved glycerine 20% v/v were added to the flask and the mixture was vortexed for a brief period. A magnet was placed inside and the mixture was heated at 60°C, while stirred at 700rpm, for a 1hour period, in a silicon oil bath. Then, the suspension was heated at 90°C for 2hours, at the same stirring. The formed suspension was left to cool down to room temperature with a rate 3°C/min and a sample of 50uL was extracted, which was diluted with 2950uL of HPLC-grade water and measured with PCS, to calculate the liposome size and polydispersity. The results for the prepared liposomes are presented in Table 2 and the size distribution by intensity, volume and number of particles with 1.8 cholesterol molar ratio is presented in Figure 2, where for each diagram, there are three curves, which in this case are very close to each other. The peak analysis areas and mean values of the three methods are given below:

Peak Analysis by intensity

| Peak | Area | Mean | Width |
|---|---|---|---|
| 1 | 100.0 | 237.5 | 349.3 |

Peak Analysis by volume

| Peak | Area | Mean | Width |
|---|---|---|---|
| 1 | 37.7 | 97.4 | 164.3 |
| 2 | 62.3 | 421.2 | 309.0 |

Peak Analysis by number

| Peak | Area | Mean | Width |
|---|---|---|---|
| 1 | 99.4 | 55.1 | 45.3 |

Table 2

| System | Molar ratio | Glycerin Concentration (% v/v) | $Z_{Ave}$ (nm) | SD | PDI | SD |
|---|---|---|---|---|---|---|
| EggPC:chol:stear | 9:0.5:0.25 | 20% | 272.2 | 13.9 | 0.602 | 0.026 |
| EggPC:chol:stear | 9:1.8:0.25 | 20% | 186.0 | 4.8 | 0.382 | 0.007 |

Example 3

Physical stability of liposomes prepared through the process of the present invention

Liposomes containing HSPC:stearylamine 9:0.25, eggPC:cholesterol:stearylamine 9:1.8:0.25 and DSPC:stearylamine 9:0.25 were developed and evaluated for their physical/colloidal stability, for around a 30-days period, by measuring their size and polydispersity with PCS.

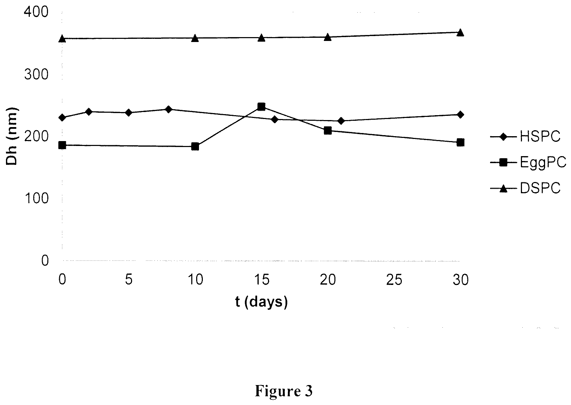

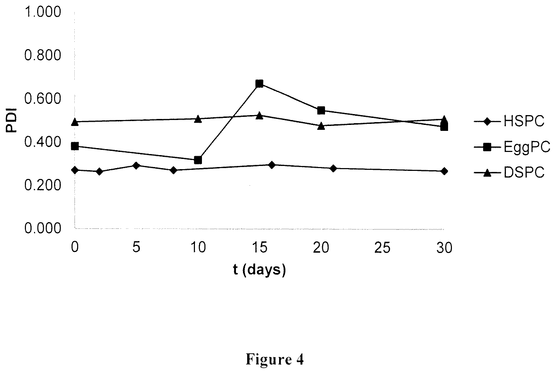

HSPC (30.0mg) and stearylamine (0.3mg) of molar ratio 9:0.25, eggPC (30.0mg), cholesterol (3.0mg) and stearylamine (0.3mg) of molar ratio 9:1.8:0.25 and DSPC (30.0mg) and stearylamine (0.3mg) of molar ratio 9:0.25 were weighted and placed inside separate large spherical flasks. 3mL of purified water with dissolved glycerine 20% v/v were added to the flask and the mixture was vortexed for a brief period. A magnet was placed inside and the mixture was heated at 60°C, while stirred at 700rpm, for a 1hour period, in a silicon oil bath. Then, the suspension was heated at 90°C for 1hour, at the same stirring. The formed suspension was left to cool down to room temperature with a rate 3°C/min and a sample of 50uL was extracted, which was diluted with 2950uL of HPLC-grade water and measured PCS, to calculate the liposome size and polydispersity. Measurements were repeated for a 30-days period, to evaluate the physical/colloidal stability of the liposomes and the results are presented in Figures 3 and 4.

Example 4

Reduction of size and size heterogeneity of large quantity of liposomes

EggPC/DPPC/HSPC:stearylamine liposomes of molar ratio 9:0.25 were prepared by mixing 2000.0/1000.0/500.0mg of EggPC/DPPC/HSPC and 5.0/10.0/20.0mg of stearylamine, adding 200/100/50mL of purified water with dissolved glycerine 10%/15%/20% v/v and heating at 90°C for 2hours, as described in Example 1. The final preparations were subjected to extrusion through polycarbonate filters of pore size 200nm. The size and polydispersity before and after extrusion were measured with PCS and are presented in Table 3.

Table 3

| System | Molar ratio | Glycerine Concentration (% v/v) | Extrusion | $Z_{Ave}$ (nm) | SD | PDI | SD |
|---|---|---|---|---|---|---|---|
| EggPC:stear | 9:0.25 | 10% | Before | 353.5 | 5.5 | 0.627 | 0.032 |
| EggPC:stear | 9:0.25 | 10% | After 1 Pass | 136.9 | 3.2 | 0.287 | 0.012 |
| EggPC:stear | 9:0.25 | 10% | After 2 Passes | 106.1 | 2.1 | 0.287 | 0.005 |
| EggPC:stear | 9:0.25 | 10% | After 5 Passes | 94.3 | 2.2 | 0.282 | 0.012 |
| EggPC:stear | 9:0.25 | 10% | After 10 Passes | 84.6 | 2.0 | 0.307 | 0.002 |
| DPPC:stear | 9:0.25 | 15% | Before | 250.2 | 11.0 | 0.402 | 0.026 |
| DPPC:stear | 9:0.25 | 15% | After 1 Pass | 155.5 | 4.2 | 0.254 | 0.032 |
| DPPC:stear | 9:0.25 | 15% | After 2 Passes | 125.9 | 2.4 | 0.182 | 0.018 |
| DPPC:stear | 9:0.25 | 15% | After 5 Passes | 127.1 | 2.3 | 0.174 | 0.004 |
| DPPC:stear | 9:0.25 | 15% | After 10 Passes | 134.6 | 2.1 | 0.204 | 0.010 |
| HSPC:stear | 9:0.25 | 20% | Before | 245.6 | 3.5 | 0.302 | 0.013 |
| HSPC:stear | 9:0.25 | 20% | After 1 Pass | 141.6 | 3.2 | 0.232 | 0.009 |
| HSPC:stear | 9:0.25 | 20% | After 2 Passes | 179.3 | 3.7 | 0.209 | 0.013 |
| HSPC:stear | 9:0.25 | 20% | After 5 Passes | 147.8 | 2.9 | 0.170 | 0.027 |
| HSPC:stear | 9:0.25 | 20% | After 10 Passes | 146.4 | 2.9 | 0.179 | 0.017 |

Example 5

Lyophilization of liposomes

The preparations of Example 1 were lyophilized and reconstituted. 500uL samples of each preparation were frozen with dry ice and acetone and immediately lyophilized at $10^{-2}$-$10^{-1}$mbar. The result of the process depended on the amount of glycerine inside the formulation. Higher amounts of glycerine led to gel-like product, while lower amounts gave powder. Reconstitution was achieved by adding the same amount of initial purified water volume q.s. 500uL. 50uL samples were diluted with 2950uL of HPLC-grade water and measured with PCS, to calculate the liposome size, poly-dispersity and $\zeta$-potential. The results are presented in Table 4.

Table 4

| System | Molar ratio | Glycerine Concentration (% v/v) | $Z_{Ave}$ (nm) | SD | PDI | SD | z-pot (mV) | SD |
|---|---|---|---|---|---|---|---|---|
| HSPC:stear | 9:0.25 | 20% | 228.3 | 1.6 | 0.294 | 0.021 | 42.3 | 0.7 |
| HSPC:stear | 9:0.25 | 15% | 304.8 | 1.3 | 0.559 | 0.016 | 49.2 | 1.9 |

Incorporation of lipophilic bioactive molecule and physical stability of complex

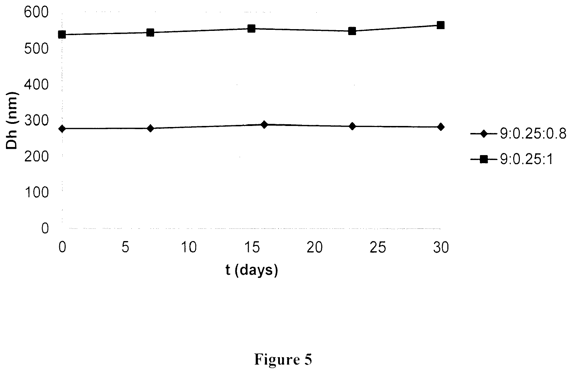

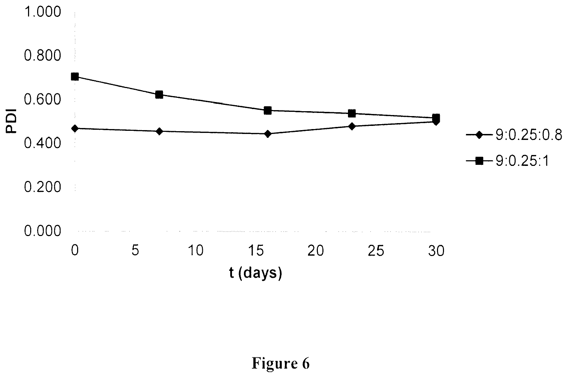

Curcumin was added to the initial lipidic mixture of Example 1, in molar ratios 9:0.25:0.8, 9:0.25:1 and 9:0.25:2 for HSPC:stearylamine:curcumin. Total lipid concentration was between 10-50mg/mL. Glycerin concentration varied between 5-20% v/v. For each case, a magnet was placed inside and the mixture was heated at 60°C, while stirred at 700rpm, for a 1hour period, in a silicon oil bath. Then, the suspension was heated at 90°C for 2hour, at the same stirring. The formed suspension was left to cool down to room temperature with a rate 3°C/min and a sample of 50uL was extracted, which was diluted with 2950uL of HPLC-grade water and measured with PCS, to calculate the liposome size and polydispersity. The results are presented in Table 7. The first and third preparations were also evaluated for their physical/colloidal stability, by repeating measurements for a 30-days period and the results are presented in Figures 5 and 6.

Table 7

| System | Molar ratio | Lipid Conc. (mg/mL) | Glycerine Conc. (% v/v) | $Z_{Ave}$ (nm) | SD | PDI | SD |
|---|---|---|---|---|---|---|---|
| HSPC:stear:curc | 9:0.25:0.8 | 10 | 20% | 277.9 | 5.3 | 0.468 | 0.056 |
| HSPC:stear:curc | 9:0.25:1 | 33 | 5% | 459.3 | 11.5 | 0.842 | 0.032 |
| HSPC:stear:curc | 9:0.25:1 | 50 | 10% | 539.4 | 5.8 | 0.706 | 0.126 |

Example 7

This example does not relate to the present invention. It shows the results of a process in which no promoter is used and heating is carried out in one step.

HSPC or eggPC (150.0mg) were weighted and placed inside a large spherical flask. It is noted that the $T_m$ is substantially different for the two lipids, namely 52 °C for HSPC and 23 °C for eggPC. 5mL of purified water with dissolved glycerine 3% v/v was added to the flask and the mixture was vortexed for a brief period. A magnet was placed inside and the mixture was heated at 60°C, while stirred at 700rpm, for a 1hour period, in a silicon oil bath. The formed suspension was left to cool down to room temperature with a rate 3°C/min and a sample of 50uL was extracted, which was diluted with 2950uL of HPLC-grade water and measured with PCS, to calculate the particle size and polydispersity. The results are presented in Table 8.

Table 8

| System | Glycerin Concentration (% v/v) | $Z_{Ave}$ (nm) | SD | PDI | SD |
|---|---|---|---|---|---|
| HSPC | 3% | 1886.8 | 397.6 | 1.000 | 0.000 |
| EggPC | 3% | 3828.0 | 1068.0 | 1.000 | 0.000 |

Example 8

This example does not relate to the present invention. It shows the results of a process in which a promoter is used and heating is carried out in one step.

HSPC (50.0mg) and stearylamine or DPPG (0.5 or 1.3mg respectively) of molar ratio 9:0.25 were weighted and placed inside a large spherical flask. 5mL of purified water with dissolved glycerine 3% v/v were added to the flask and the mixture was vortexed for a brief period. A magnet was placed inside and the mixture was heated at 60°C, while stirred at 700rpm, for a 1hour period, in a silicon oil bath. The formed suspension was left to cool down to room temperature with a rate 3°C/min and a sample of 50uL was extracted, which was diluted with 2950uL of HPLC-grade water and measured with PCS, to calculate the particle size, polydispersity and ζ-potential. The results are presented in Table 9.

Table 9

| System | Molar ratio | Glycerin Concentration (% v/v) | $Z_{Ave}$ (nm) | SD | PDI | SD | z-pot (mV) | SD |
|---|---|---|---|---|---|---|---|---|
| HSPC:stear | 9:0.25 | 3% | 702.3 | 31.7 | 1.000 | 0.000 | 15.7 | 9.3 |
| HSPC:DPPG | 9:0.25 | 3% | 825.6 | 18.0 | 1.000 | 0.000 | -29.2 | 1.2 |

Example 9

This example does not relate to the present invention. It shows a process in which the second heating step is carried out at the same temperature as that of the first heating step.

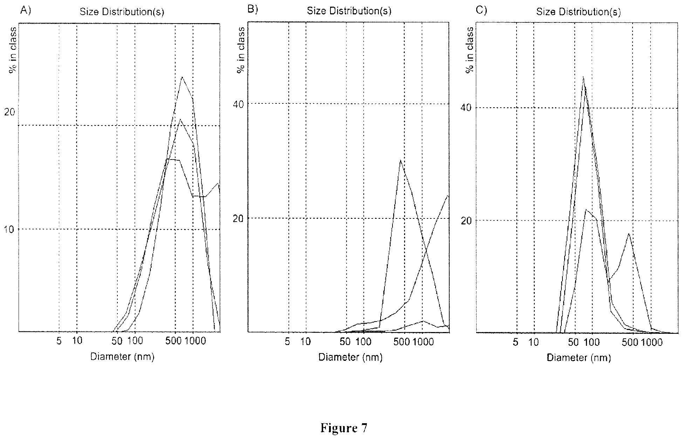

HSPC (30.0mg) and stearylamine (0.3mg) of molar ratio 9:0.25 were weighted and placed inside a large spherical flask. 3mL of purified water with dissolved glycerine 20/15/10/5% v/v were added to the flask and the mixture was vortexed for a brief period. A magnet was placed inside and the mixture was heated at 60°C, while stirred at 700rpm, for a 2hour period, in a silicon oil bath. The formed suspension was left to cool down to room temperature with a rate 3°C/min and a sample of 50uL was extracted, which was diluted with 2950uL of HPLC-grade water and measured with PCS, to calculate the particle size and polydispersity. Then, the suspension was heated at 60°C for another 1hour, at 700rpm and after cooling, another sample of 50uL was measured. The results are presented in Table 10 and the size distribution by intensity, volume and number of particles with 20% glycerin is presented in Figure 7, where for each diagram, there are three curves. The peak analysis areas and mean values of the three methods are given below:

Peak Analysis by intensity

| Peak | Area | Mean | Width |
|---|---|---|---|
| 1 | 79.7 | 654.6 | 1359.4 |
| 2 | 20.3 | 8799.7 | 5920.4 |

Peak Analysis by volume

| Peak | Area | Mean | Width |
|---|---|---|---|
| 1 | 5.7 | 899.9 | 523.3 |
| 2 | 94.3 | 9825.2 | 10972.1 |

Peak Analysis by number

| Peak | Area | Mean | Width |
|---|---|---|---|
| 1 | 100.0 | 90.3 | 95.4 |

Table 10

| System | Molar ratio | Glycerin Concentration (% v/v) | Hours at 60°C | $Z_{Ave}$ (nm) | SD | PDI | SD |
|---|---|---|---|---|---|---|---|
| HSPC:stear | 9:0.25 | 20% | 2 | 498.6 | 33.0 | 0.820 | 0.134 |
| HSPC:stear | 9:0.25 | 20% | 3 | 484.1 | 37.5 | 0.814 | 0.188 |
| HSPC:stear | 9:0.25 | 15% | 2 | 615.8 | 26.1 | 0.797 | 0.351 |
| HSPC:stear | 9:0.25 | 15% | 3 | 522.4 | 31.5 | 0.768 | 0.227 |
| HSPC:stear | 9:0.25 | 10% | 2 | 576.4 | 28.2 | 0.825 | 0.185 |
| HSPC:stear | 9:0.25 | 10% | 3 | 539.6 | 120.3 | 0.875 | 0.126 |
| HSPC:stear | 9:0.25 | 5% | 2 | 724.9 | 12.0 | 1.000 | 0.000 |
| HSPC:stear | 9:0.25 | 5% | 3 | 806.0 | 60.6 | 1.000 | 0.000 |

The composition of the first six lipidic vehicles of Table 10 is the same as the composition of the vehicles of Table 1 (Example 1). The process of this Example differs from the process of Example 1 in that there is no temperature increase in the second heating step. The results show that the lack of temperature leap negatively affects the properties of the obtained lipidic vehicles.

**Claims**

1. A process for the production of lipidic vehicles comprising the following steps:

a) providing a mixture of an amphiphilic lipid and a promoter in a liquid medium comprising water and a liquid polyol, wherein the promoter is selected from dipalmitoylphosphatidylglycerol, dioleoyltrimethylammoniumpropane, cholesterol, fatty acids, fatty acid amines, poloxamers, polyethylene glycol with molecular weight higher than 600 and mixtures thereof and the liquid polyol is selected from glycerine, propylene glycol, polyethylene glycol with molecular weight lower than 600 and mixtures thereof,
b) stirring and heating the mixture in a first heating step at 30-80°C,
c) stirring and heating the mixture in a second heating step at a temperature 10-50°C higher than the temperature of the first heating step and
d) allowing the mixture to cool down to room temperature.

2. A process for the production of lipidic vehicles, according to claim 1, wherein the lipidic vehicles are selected from liposomes and niosomes.

3. A process for the production of lipidic vehicles, according to claim 1 or 2, wherein the lipidic vehicles are liposomes.

4. A process for the production of lipidic vehicles, according to any one of claims 1 to 3, wherein the amphiphilic lipid is selected from phospholipids, ether lipids, cardiolipins, sphingolipids, ionic or non-ionic surfactants, adjuvant lipids, and mixtures thereof.

5. A process for the production of lipidic vehicles, according to any one of claims 1 to 4, wherein the amphiphilic lipid is selected from hydrogenated soy phosphatidylcholine, egg phosphatidylcholine, dipalmitoylphosphatidylcholine, distearoylphosphatidylcholine, dimyristoylphosphatidylcholine, dioleoylphosphatidylcholine, dipalmitoylphosphatidylethanolamine, distearoylphosphatidylethanolamine, dioleoylphosphatidylethanolamine, fatty acid- or headgroup-modified phospholipid, ceramides, dimethyldioctadecylammonium, sorbitan lipids, polysorbates, and mixtures thereof.

6. A process for the production of lipidic vehicles, according to any one of claims 1 to 5, wherein the promoter is stearylamine.

7. A process for the production of lipidic vehicles, according to any one of claims 1 to 6, wherein the liquid polyol is glycerine.

8. A process for the production of lipidic vehicles, according to any one of claims 1 to 7, wherein the concentration of the liquid polyol is up to 30% v/v of the liquid medium.

9. A process for the production of lipidic vehicles, according to any one of claims 1 to 8, wherein the concentration of the liquid polyol is 10% - 30% v/v of the liquid medium.

10. A process for the production of lipidic vehicles, according to any one of claims 1 to 9, wherein the temperature of the mixture of the second heating step is 20-40°C higher than the temperature of the first heating step.

11. A process for the production of lipidic vehicles, according to any one of claims 1 to 10, wherein in step d) the mixture is cooled down at a rate not greater than 5°C/min.

12. A process for the production of lipidic vehicles, according to any one of claims 1 to 11, wherein the process further comprises adding to the liquid medium a bioactive ingredient.

13. A process for the production of lipidic vehicles, according to any one of claims 1 to 12, wherein the process further comprises subjecting the mixture after step d) to lyophilization.

**Patentansprüche**

1. Verfahren zur Herstellung von Lipidvehikeln aufweisend die folgenden Schritte:

a) Bereitstellen einer Mischung aus einem amphiphilen Lipids und einem Promotor in einem flüssigen Medium, das Wasser und ein flüssiges Polyol aufweist, wobei der Promotor aus Dipalmitoylphosphatidylglycerin, Dioleoyltrimethylammoniumpropan, Cholesterin, Fettsäuren, Fettsäureaminen, Poloxameren, Polyethylenglykol mit Molgewicht höher als 600 und Mischungen davon ausgewählt ist and das flüssige Polyol aus Glycerin, Propylenglykol, Polyethylenglykol mit Molgewicht niedriger als 600 und Mischungen davon ausgewählt ist,
b) Rühren und Erhitzen der Mischung in einem ersten Erhitzungsschritt bei 30-80°C.
c) Rühren und Erhitzen der Mischung in einem zweiten Erhitzungsschritt bei einer Temperatur, die 10-50°C höher als die Temperatur des ersten Erhitzungsschritts ist, und
d) Abkühlen lassen der Mischung auf Raumtemperatur.

2. Verfahren zur Herstellung von Lipidvehikeln nach Anspruch 1, wobei die Lipidvehikel aus Liposomen und Niosomen ausgewählt sind.

3. Verfahren zur Herstellung von Lipidvehikeln nach Anspruch 1 oder 2, wobei die Lipidvehikel Liposomen sind.

4. Verfahren zur Herstellung von Lipidvehikeln nach einem der Ansprüche 1 bis 3, wobei das amphiphile Lipid aus Phospholipiden, Etherlipiden, Cardiolipinen, Sphingolipiden, ionischen oder nichtionischen Tensiden, Adjuvanslipiden und Mischungen davon ausgewählt ist.

5. Verfahren zur Herstellung von Lipidvehikeln nach einem der Ansprüche 1 bis 4, wobei das amphiphile Lipid aus hydriertem Sojaphosphatidylcholin, Eiphosphatidylcholin, Dipalmitoylphosphatidylcholin, Distearoylphosphatidylcholin, Dimyristoylphosphatidylcholin, Dioleoylphosphatidylcholin, Dipalmitoylphosphatidylethanolamin, Distearoylphosphatidylethanolamin, Dioleoylphosphatidylethanolamin, Fettsäure- oder Kopfgruppe-modifiziertes Phospholipid, Ceramiden, Dimethyldioctadecylammonium, Sorbitanlipiden, Polysorbaten und Mischungen davon ausgewählt ist.

6. Verfahren zur Herstellung von Lipidvehikeln nach einem der Ansprüche 1 bis 5, wobei der Promotor Stearylamin ist.

7. Verfahren zur Herstellung von Lipidvehikeln nach einem der Ansprüche 1 bis 6, wobei das flüssige Polyol Glycerin ist.

8. Verfahren zur Herstellung von Lipidvehikeln nach einem der Ansprüche 1 bis 7, wobei die Konzentration des flüssigen Polyols bis zum 30% v/v des flüssigen Mediums beträgt.

9. Verfahren zur Herstellung von Lipidvehikeln nach einem der Ansprüche 1 bis 8, wobei die Konzentration des flüssigen Polyols 10% - 30% v/v des flüssigen Mediums beträgt.

10. Verfahren zur Herstellung von Lipidvehikeln nach einem der Ansprüche 1 bis 9, wobei die Temperatur der Mischung des zweiten Erhitzungsschritts 20-40°C höher als die Temperatur des ersten Erhitzungsschritts ist.

11. Verfahren zur Herstellung von Lipidvehikeln nach einem der Ansprüche 1 bis 10, wobei in Schritt d) die Mischung mit einer Rate von nicht mehr als 5 °C/min abgekühlt wird.

12. Verfahren zur Herstellung von Lipidvehikeln nach einem der Ansprüche 1 bis 11, wobei das Verfahren ferner das Hinzufügen eines bioaktiven Bestandteils zu dem flüssigen Medium umfasst.

13. Verfahren zur Herstellung von Lipidvehikeln nach einem der Ansprüche 1 bis 12, wobei das Verfahren ferner nach Schritt d) das Unterziehen der Mischung einer Gefriertrocknung umfasst.

**Revendications**

1. Procédé de production des véhicules lipidiques comprenant les étapes suivis pour :

   a) fournir un mélange d'un lipide amphiphile et d'un promoteur dans un moyen liquide comprenant de l'eau et d'un polyol liquide, dans lequel le promoteur est choisi parmi le dipalmitoylphosphatidylglycérol, le dioléoyltriméthylammoniumpropane, le cholestérol, les acides gras, les amines d'acides gras, les poloxamères, le polyéthylèneglycol de masse moléculaire en poids plus de 600 et leurs mélanges et dans lequel le polyol liquide est choisi parmi la glycérine, le propyléneglycol, le polyéthylèneglycol de masse moléculaire en poids plus de 600 et leurs mélanges,
   b) agiter et réchauffer le mélange dans une première étape d'échauffement à 30-80°C,
   c) agiter et réchauffer le mélange dans une deuxième étape d'échauffement à une température supérieure à 10-50°C que la température de la première étape d'échauffement et
   d) permettre le mélange à refroidir jusqu'à la température ambiante.

2. Procédé de production des véhicules lipidiques, selon la revendication 1, dans lequel les véhicules lipidiques sont choisis parmi les liposomes et les niosomes.

3. Procédé de production des véhicules lipidiques, selon la revendication 1 ou 2, dans lequel les véhicules lipidiques sont des liposomes.

4. Procédé de production des véhicules lipidiques, selon l'une quelconque des revendications 1 à 3, dans lequel le lipide amphiphile est choisi parmi les phospholipides, les éthers lipidiques, les cardiolipines, les sphingolipides, les agents de surface ioniques ou non-ioniques, les lipides des adjuvants, et leurs mélanges.

**5.** Procédé de production des véhicules lipidiques, selon l'une quelconque des revendications 1 à 4, dans lequel le lipide amphiphile est choisi parmi la phosphatidylcholine de soja hydrogénée, la phosphatidylcholine de l'œuf, la dipalmitoylphosphatidylcholine, la distéaroylphosphatidylcholine, la dimyristoylphosphatidylcholine, la dioléoylphosphatidylcholine, la dipalmitoylphosphatidyléthanolamine, la distéaroylphosphatidyléthanolamine, la dioléoylphosphatidyléthanolamine, le phospholipide à acides gras ou à têtes polaires modifies, les céramides, le diméthyldioctadécylammonium, les lipides de sorbitane, les polysorbates, et leurs mélanges.

**6.** Procédé de production des véhicules lipidiques, selon l'une quelconque des revendications 1 à 5, dans lequel le promoteur est de la stéarylamine.

**7.** Procédé de production des véhicules lipidiques, selon l'une quelconque des revendications 1 à 6, dans lequel le polyol liquide est de la glycérine

**8.** Procédé de production des véhicules lipidiques, selon l'une quelconque des revendications 1 à 7, dans lequel la concentration du polyol liquide est jusqu'à 30% titre volumique du moyen liquide.

**9.** Procédé de production des véhicules lipidiques, selon l'une quelconque des revendications 1 à 8, dans lequel la concentration du polyol liquide est 10% - 30% titre volumique du moyen liquide.

**10.** Procédé de production des véhicules lipidiques, selon l'une quelconque des revendications 1 à 9, dans lequel la température de la deuxième étape d'échauffement est supérieure à 20-40°C que la température de la première étape d'échauffement.

**11.** Procédé de production des véhicules lipidiques, selon l'une quelconque des revendications 1 à 10, dans lequel l'étape d) consiste en refroidir le mélange à une vitesse non supérieure à 5°C/min.

**12.** Procédé de production des véhicules lipidiques, selon l'une quelconque des revendications 1 à 11, dans lequel le procédé consiste en outre en ajoutant un ingrédient bioactif dans le moyen liquide.

**13.** Procédé de production des véhicules lipidiques, selon l'une quelconque des revendications 1 à 12, dans lequel le procédé consiste en outre en appliquant de la lyophilisation au mélange après l'étape d).

A) Size Distribution(s)

B) Size Distribution(s)

C) Size Distribution(s)

**Figure 1**

Figure 2

Figure 3

**Figure 4**

Figure 5

Figure 6

**Figure 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100239521 A
- WO 2005084641 A
- US 2008274172 A1

### Non-patent literature cited in the description

- **D. PAPAHADJOPOULOS ; J.C. WATKINS.** Phospholipid model membrane, Permeability properties of hydrated liquid crystals. *Biochim. Biophys. Acta,* 1967, vol. 135, 639
- **J. MILHAUD.** New insights into water-phospholipid model membrane interactions. *Biochim. Biophys. Acta,* 2004, vol. 1663, 19
- **D.D. LASIC et al.** Spontaneous vesiculation. *Adv. Colloid. Interface Sci.,* 2001, vol. 89-90, 337
- **C. DEMETZOS.** Pharmaceutical Nanotechnology: Fundamentals and practical applications. Springer, 2016
- **V. GUIDA.** Thermodynamics and kinetics of vesicles formation processes. *Adv. Colloid. Interface Sci.,* 2010, vol. 161 (1-2), 77
- **C. PUGLIA ; F. BONINA.** Lipid nanoparticles as novel delivery systems for cosmetics and dermal pharmaceuticals. *Expert Opin. Drug Deliv.,* 2012, vol. 9, 429
- **J.N. ISRAELACHVILI et al.** Theory of self-assembly of hydrocarbon amphiphiles into micelles and bilayers. *J. Chem. Soc., Far. Trans. 2: Mol. Chem. Phys.,* 1976, vol. 72, 1525
- **R. NAGARAJAN.** Molecular Packing Parameter and Surfactant Self-Assembly: The Neglected Role of the Surfactant Tail. *Langmuir,* 2002, vol. 18, 31
- **D. PAPAHADJOPOULOS et al.** Sterically stabilized liposomes: Improvements in pharmacokinetics and antitumor therapeutic efficacy. *Proc. Natl. Acad. Sci. USA,* 1991, vol. 88, 11460
- **M. RIAZ.** Liposome Preparation Method. *J. Pharm. Sci.,* 1996, vol. 19 (65
- **Y.P. PATIL ; S. JADHAV.** Novel methods for liposome preparation. *Chem Phys. Lipids.,* 2014, vol. 177, 8-18
- **M. RIAZ.** Liposome Preparation Method. *J. Pharm. Sci.,* 1996, vol. 19, 65